# EUROPEAN PATENT SPECIFICATION

(11) **EP 1 030 734 B1**
(45) Date of publication and mention of the grant of the patent: **06.08.2003**
(21) Application number: 98952882.3
(22) Date of filing: 10.11.1998
(51) Int. Cl.: B01J 13/08, A61K 7/16, A61K 7/00, A23L 1/22, A23L 1/27

(54) **ENCAPSULATE OF ACTIVE MATERIAL IN ALGINATE MATRIX**
VERKAPSELTES AKTIVES MATERIAL IN EINER ALGINATMATRIX
ENCAPSULAT D'UN MATERIAU ACTIF DANS UNE MATRICE D'ALGINATE

(30) Priority: 10.11.1997 EP 97308991
(43) Date of publication of application: 30.08.2000
(73) Proprietor: QUEST INTERNATIONAL B.V., 1411 GP Naarden (NL)
(72) Inventor: NESS, Jeremy, Nicholas, Ashford, Kent TN25 4DQ (GB); GOODALL, Marcus, James, Canterbury, Kent CT4 7AB (GB); MOYSEN, Ruth, Constance, Surrey RH8 0LG (GB)
(74) Representative: Matthews, Heather Clare
(86) International application number: GB9803345
(87) International publication number: WO99024159

(56) References cited:
- EP-A- 0 391 803
- EP-A- 0 747 037
- WO-A-96/40069
- FR-A- 1 529 576
- GB-A- 929 403
- GB-A- 2 237 574
- US-A- 5 498 439
- DATABASE WPI Section Ch, Week 8010 Derwent Publications Ltd., London, GB; Class A11, AN 80-17311C XP002059394 & JP 55 011029 A (KIBUN KK) , 25 January 1980

## Description

### Field of the Invention

This invention concerns toothpastes incorporating encapsulates.

### Background to the Invention

Encapsulation is a well known technique for presenting active materials such as flavour, fragrance, colouring materials etc. Encapsulate systems fall into two main types: those in which the active material is surrounded by a wall or barrier; and those in which the active material is encapsulated in the matrix of a material. A common form of matrix encapsulate is based on a matrix of cross-linked alginate gels.

Alginates are naturally occurring colloids which are normally extracted from brown seaweed (Pnaeophycea). which are used mainly in the form of sodium salts. Sodium alginates give very viscous solutions with water and can be irreversibly cross-linked to form thermally stable gels by interaction with divalent or trivalent metal ions, typically calcium ions. Using this interaction, active materials may be encapsulated or entrapped in an alginate gel matrix. The colloid is principally alginic acid which is made up of three kinds of polymer segments, one segment containing D-mannuronic acid units; a second segment containing L-guluronic acid; and a third segment containing alternating D-mannuronic and L-guluronic acid residues, joined by 1:4 glycosidic linkages. The proportions of the three polymer segments in alginic acid samples give rise to different properties, for example, the alginate from Laminaria hyperborea, with a large percentage of polyguluronate segments, forms rigid, brittle gels which tend to undergo syneresis.

There is a large amount of literature in the area of dental flavour encapsulation, not only to provide flavour stability but also to develop a unique flavour delivery system which provides a perceivable sensory effect and longevity of the flavour in the mouth. To this end, numerous systems have been tested but there are currently no toothpastes in the market place which utilise a flavour encapsulation system.

US 4389419 (Lim) concerns capsules of oil soluble nutrients such as vitamins which are formed by making an emulsion of sodium alginate with the nutrient and with optional alcohol-insoluble polysaccharide filler or extender such as dextran, sodium carboxy methyl cellulose, methyl cellulose, dextrins and some soluble starches and sodium carboxy cellulose, and adding the emulsion as droplets to an alcoholic solution containing calcium chloride to give solid capsules. The resulting capsules or beads are dried. The wall of the capsule comprises the matrix of water-insoluble multivalent cation-containing gel and filler, and within the matrix is a plurality of compartments containing oil droplets shielded from atmospheric exposure. Column 2 lines 48 to 50 state that advantageously no emulsifying agents need to be employed since the alginate (which is present at high levels) effectively serves this function. No uses of the capsules are specified.

EP 0202819A (Warner Lambert) discloses encapsulates formed by mixing alginate with active ingredient. particularly to produce a dispersion, and crosslinking the alginate by exposure to divalent cations, for example by spraying the dispersion into calcium chloride to produce particles eg up to 10 micron in diameter. The resulting particles are dried. The active ingredients may be flavouring, colouring agent, fragrance etc. The encapsulates may be used particularly in chewing gum, but also in confectionary, foods, pharmaceuticals, toothpaste etc. No details are given of toothpaste formulations.

EP 0747037A (Sara Lee) discloses toothpaste including microspheres 0.1-4mm in diameter of zinc-stabilised alginate. The microspheres can contain ingredients such as enzymes, abrasives, flavour, colour, bactericides etc. The microspheres are prepared by adding droplets of alginate solution to a zinc salt solution such as zinc chloride. The alginate solution preferably includes a surfactant such as polyethylene glycol (PEG) or ethoxylated glycerol, for unspecified purpose. The materials are not in the form of an emulsion.

### Summary of the Invention

In one aspect the present invention provides a water-based toothpaste comprising: a hydrated dental flavour encapsulate comprising an emulsion or dispersion of dental flavour in an alginate matrix; an anionic surfactant; and an electrolyte, wherein the electrolyte comprises magnesium sulphate.

The present invention provides a flavour release system, which is incorporated into water-based toothpastes, is stable on storage yet releases during brushing in the mouth. The invention may also provide for a visual effect in the toothpaste, contributing to its appeal, which can enhance both flavour intensity and longevity. There is also the opportunity to deliver novel flavour effects e.g. change of flavour on brushing.

It has been found that by having the dental flavour in the form of an emulsion or dispersion, and by having the encapsulates in hydrated form, benefits in terms of product stability during storage and use can be obtained. In particular, the properties of the dental flavour are less likely to deteriorate with time and the encapsulates are more stable in possibly hostile environments of use.

As well as dental flavour, the encapsulate may optionally contain one or more active materials selected from a wide range of different materials including, for example, flavouring materials, including food flavours, fragrance materials, colouring materials, enzymes, therapeutic agents such as anti-bacterial agents, anti-caries agents, etc. The active material is preferably substantively insoluble in water, and/or preferably sufficiently large (eg preferably having a molecular weight of at least 5000) to be retained by the alginate. Suitable materials are well known. A mixture of active materials may be used.

The dental flavour may be present in relatively large amounts, typically constituting up to 60% by weight of the total weight of the encapsulates. The dental flavour is typically present in an amount in the range 10 to 60%, preferably 20 to 60%, eg 25 to 30% by weight of the total weight of the encapsulate. References to the total weight of the encapsulate mean the weight of all encapsulate ingredients, including water etc.

The alginate is conveniently in the form of a sodium salt, and is preferably an alginate having a high percentage of polyguluronic acid units (known as high G alginates) as these produce encapsulates with improved stability on storage. Suitable alginates are commercially available, and include, for example, the high G alginate Manugel DMB (Manugel is a Trade Mark) supplied by Nutrasweet Kelco. The aforementioned alginates provide a strong structure, which can for example be incorporated into a base using standard toothpaste mixing techniques, and which keeps the rigidity of the capsules on storage.

The alginate is typically present in an amount of up to about 5% by weight of the total weight of the encapsulate. Good results have been obtained at lower levels, eg constituting about 1% or less of the total weight of the encapsulate.

The weight ratio of dental flavour to alginate is preferably in the range 5:1 to 300:1, more preferably 10:1 to 100:1, most preferably 20:1 to 60:1.

Where an active material is a solid, eg zinc salts such as zinc citrate trihydrate, the alginate and active material form a dispersion.

When the dental flavour is a liquid, the alginate and dental flavour form an emulsion, and are preferably formed into an emulsion by use of an emulsifying agent. The emulsifying material is preferably a water soluble polysaccharide, with a molecular weight of greater than 10,000. For example hydroxy cellulosic materials, a polysaccharide derived from the monosaccharide glucose, such as hydroxyethyl cellulose (HEC), hydroxypropyl cellulose (HPC) and hydroxypropylmethyl cellulose, or other polysaccharides containing different sugar monosacchandes where some of the primary alcohol groups of the polysaccharide have been oxidised to form uronic acid, for example D-glucuronic acid formed by the oxidation of the primary alcohol groups of glucose. Other examples include D-mannuronic acid and D-galacturonic acid. Such polysaccharides and their derivatives include Pectins, gum Tragacanth, alginates, propylene glycol alginate, gum Arabic and gum Ghatti. Combinations of different gums can also be used to achieve superior emulsion stability, such as the combination of propylene glycol alginate and pectins. These materials, particularly propylene glycol alginate, have good emulsifying properties and result in encapsulates with good stability.

The emulsifying agent preferably has a molecular weight (weight average) greater then 10,000, more preferably greater then 25,000, and particularly less than 2,000,000, and especially less than 1,000,000.

The emulsifying agent is typically present in an amount in the range from 0.2 to 5%, more preferably 0.5 to 2% and particularly 0.5 to 1% by weight of the total weight of the encapsulate.

The encapsulates preferably contain little or no materials which would act to solubilise the dental flavour. Such as most surfactants with a molecular weight of less than 5000, possibly resulting in leaching of the dental flavour from the encapsulates over time. Some common surfactants such as Tween 20 (polyoxyethylene (20) sorbitan monolaurate ex ICI - Tween 20 is a Trade Mark) may have this effect, and so preferably should be avoided.

The balance of the encapsulate ingredients is usually water, preferably distilled water.

Alginate encapsulation is a known technique, and methods for producing encapsulates for use in water-based toothpastes in accordance with the invention, eg using techniques discussed in the prior art mentioned above, are known to those skilled in the art.

A preferred method of producing hydrated encapsulates of dental flavour, comprises forming an emulsion or dispersion of alginate and dental flavour, and causing cross-linking of the alginate to produce hydrated encapsulates.

the method conveniently involves mixing alginate, dental flavour, emulsifying agent and water to produce an aqueous emulsion, eg using high shear mixing, and contacting droplets of the emulsion with an aqueous solution of those divalent or trivalent metal ions known to form insoluble gels, such as calcium and zinc, eg by spraying the emulsion into calcium chloride solution. Therefore, by preparing an emulsion of an alginate solution and dental flavour and adding this, drop wise, into a calcium chloride solution, spherical encapsulates are formed. Encapsulates of generally spherical form are produced virtually instantaneously by the known irreversible cross-linking reaction of alginate. If a chloride solution is used, the encapsulates are then preferably washed in water to remove chloride ions as these have a bitter taste, with the encapsulates then being suspended in water. Where other counterions such as lactate that do not have an unpleasant taste are used, washing is not required. The encapsulates should preferably be stored in hydrated form prior to use. Freeze drying or other drying techniques should preferably be avoided. The capsules can then be dosed into a toothpaste and rupture, releasing the flavour, on brushing.

By varying factors including emulsion viscosity, speed of spraying, droplet travel distance, droplet size etc in known manner, encapsulates of desired size can be produced. For most purposes, encapsulates having a diameter in the range 0.1 to 3mm, preferably 0.3 to 1mm, are suitable.

Encapsulate properties may also be affected to some extent by the time of contact with the calcium chloride or similar solution.

A wide range of dental flavours may be used which are well known to those skilled in the art and are commonly mint-based. A mixture of dental flavours may be used.

The dental flavour encapsulate conveniently also comprises an orally-compatible colouring material in the form of a pigment or oil soluble colour, eg titanium oxide for white colour, for aesthetic reasons to produce an attractive coloured product instead of a clear or colourless product.

The encapsulates may additionally include active materials such as triclosan and zinc salts, eg zinc citrate trihydrate.

Encapsulates for use in water-based toothpastes in accordance with the invention can be stable in the water-based toothpastes on storage yet release the dental flavour on brushing with no adverse mouthfeel effects, in a way that has not hitherto been possible. The toothpaste can include one or more non-ionic surfactants and/or possibly one or more amphoteric surfactants.

Any non-ionic surfactant can be used for this purpose, including, for example, polyoxyethylene alkyl ethers, polyoxyethylene alklaryl ethers, polyoxyethylene-polyoxypropylene alkyl ethers, polyoxyethylene-polyoxypropylene block copolymers, sucrose fatty acid esters, glycerol fatty acid esters, propylene glycol fatty acid esters, sorbitan fatty acid esters, alkylglycoside fatty acid esters, polyoxyethylene sucrose fatty acid esters, polyoxyethylene glycol fatty acid esters, polyoxyethylene sorbitan fatty acid esters, alkylglycosides, fatty acid monoethanolamide, polyoxyethylene fatty acid monoethanolamide, polyoxyetheylene fatty acid diethanolamide, polyoxyethylene castor oils, poloxyethylene hydrogenated castor oils, and polyoxytheylene beeswax derivatives. A mixture of non-ionic surfactants may be used.

Amphoteric surfactants, such as cocoamidopropyl betaine, are also suitable for use in toothpastes.

The encapsulates for use in a water-based toothpaste in accordance with the invention are very rigid, post production, and can therefore be mixed into the toothpaste base using standard mixing techniques, and become weaker during storage in the product due to ion exchange reactions and loss of a proportion of the crosslinking ions to the non-ionic surfactant, of present, and any sequestrant in the product. However, the capsules remain intact during storage and give good mouthfeel effects on brushing, requiring little shear to release the dental flavour.

The capsules can become unstable in the presence of anionic surfactants due to the removal of most of the crosslinking ions from the capsule system as the presence of any anion which will remove divalent ions by precipitation or sequestration will tend to solubilise the alginate. In the case of the capsules for use in a water-based toothpaste in accordance with this invention, this results in flavour leaching and/or capsule dissolution since the corresponding calcium salts of anionic surfactants tend to be insoluble in water, therefore, precipitating out and become ineffective and cause clouding of the product.

However, sodium lauryl sulphate (SLS) is commonly used in toothpaste due to its high foaming efficiency, which can be difficult to achieve using non-ionic surfactants, and therefore, as the encapsulates may be used in water based toothpaste containing e.g. SLS, a stabilising system is needed to reduce the amount of free monomer surfactant in the product. The preferred stabilising system for the encapsulates is an electrolyte/counterion which decreases the critical micelle concentration of the surfactant by partially neutralising the anionic charge, decreasing the charge density. The electrolyte is particularly magnesium sulphate, which can be added to the product formulation. The concentration of the electrolyte is preferably in the range from 0.2 to 5%, more preferably 0.5 to 3%, and particularly 1 to 2% by weight of the total weight of the toothpaste.

The encapsulate stability in anionic surfactants is dramatically increased by the inclusion of, for example, 2% magnesium sulphate which not only improves the stability of the capsules by reducing the leaching of dental flavour but also improves the clarity of the product as less of the surfactant is precipitated out as calcium lauryl sulphate. The encapsulates may also decrease in size as they undergo their natural syneresis process, which can be regarded as a continuation of the process of gelation, and in consequence, they become more rigid. Thus. the encapsulates require more shear to rupture and release the entrapped active, however, this does not impart any adverse mouthfeel effects.

Most electrolytes appear to increase the stability of the encapsulates to some extent, for example tests carried out with sodium monofluorophosphate and sodium hydrogen carbonate showed that both the electrolytes increased the stability of the capsules in a 2% SLS solution when compared to a capsule sample containing no electrolyte. However, none of the electrolytes tested achieved the level of stability gained from incorporating magnesium sulphate. The use of magnesium sulphate also has the added advantage of being orally acceptable.

Another method of increasing capsule stability is to add a further additive to the capsule formulation to increase the barrier properties of the system. These include gums which are crosslinked by other ions such as Carrageenan (cross-linked with calcium and potassium ions) or Gellan gum (cross-linked with potassium, sodium, calcium and magnesium ions) or the addition of colloidal silica to the formulation, which not only increases the barrier properties but also acts as an emulsifier. Alternatively the addition of a gum which interacts with the alginate itself can be added to the formulation such as sodium carboxymethyl cellulose. These methods are effective at preventing the dissolution of the capsule in some anionic formulations, however, they only delay the leaching process.

The toothpaste may be otherwise of generally conventional formulation, eg as disclosed in EP 0747037. Additional possibilities not mentioned in EP 0747037 include the following:
Additional humectant - xylitol
Additional thickener - xanthan gum
Any suitable flavour - can include both natural and/or synthetic
Additional sweetening agents - aspartame, acesultame K, talin
Additional anti-plaque agent -cetyl pyridinium chloride
Use of peroxides and other whitening agents
pH range 5.5 to 9.0

The encapsulates can be readily incorporated into standard toothpaste bases using conventional toothpaste mixing techniques.

The use of high weight ratios of dental flavour to alginate, as noted above, gives a cost-effective toothpaste with good flavour release and mouthfeel properties in use.

As noted above, encapsulates can be stable in toothpastes so that the dental flavour, remains encapsulated in the toothpaste on storage. The encapsulates will normally be colourless and so not be visually apparent in the toothpaste, however if the encapsulates include a colouring material or dye they will be visible in the toothpaste possibly providing a pleasing visual effect that will be readily apparent if the toothpaste is presented in a transparent package.

The alginate encapsulates weaken and rupture easily, eg on dispensing or use, while nevertheless remaining intact on storage. If rupturing of coloured encapsulates occurs on dispensing, eg squeezing the toothpaste from a tube, this may result in formation of stripes in the toothpaste, which again may be visually pleasing. The encapsulates will in any event rupture on use, eg on brushing, with consequential release of contents. This opens the possibility of creating novel flavour effects, such as change of flavour on brushing.

Comparative tests have shown that a given amount of flavouring material in a toothpaste produces a stronger effect when in the form of encapsulates in accordance with the invention, thus providing possibilities for reducing flavouring material usage in toothpaste, with consequential cost savings, without loss of fiavour effects.

The invention will be further described, by way of illustration, in the following Examples.

### Example 1

Dental flavour encapsulates for use in a water-based toothpaste in accordance with the invention were prepared by mixing using a high shear Silverson mixer the following ingredients (amounts specified in % by weight) to form an emulsion.

| | |
|---|---|
| Sodium Alginate (Manugel DMB ex Nutrasweet Kelco) | 0.8% |
| HEC (z*Natrosol 250LR ex Aqualon) | 0.5% |
| Distilled Water | 74.7% |
| Dental Flavour A (Peppermint) | 25.0% |

| | |
|---|---|
| * Natrosol is a Trade Mark. | |

The composition of dental flavour A is as follows:

| | Weight % |
|---|---|
| 1-methoxy-4-propenylbenzene | 8.6 |
| Clove Bud Oil | 0.3 |
| Eucalyptol | 2.7 |
| Lemon Oil | 0.5 |
| 3-hydroxy-2-methyl-4-pyrone | 0.1 |
| Menthol laevo | 28.7 |
| Orange Oil | 0.2 |
| Pepper Black Oil | 0.1 |
| Peppermint Oil | 58.8 |
| Total | 100.0 |

The emulsion was sprayed as droplets into a reservoir of aqueous calcium chloride, resulting in irreversible cross-linking of the alginate to produce generally spherical encapsulates. The encapsulates were removed from the calcium chloride solution after an appropriate residence time (approximately sixty seconds) to produce encapsulates having desired features. The encapsulates were washed in distilled water to remove free chloride ions that would cause a bitter taste in the final product, and suspended in distilled water for storage. The encapsulates had a diameter of about 1mm in the present case. obtained in known manner by regulating factors including emulsion viscosity, spray pump speed, spray nozzle size and distance from spray nozzle to calcium chloride solution.

The encapsulates can be stored in hydrated form for extended periods of time (at least 6 months) without significant loss of flavour. In contrast, freeze-drying of the encapsulates resulted in substantial and immediate loss of flavour.

### Example 2

Dental flavour encapsulates were prepared by making an emulsion of the following ingredients using a Silverson mixer.

| | Weight % |
|---|---|
| Sodium Alginate (Manugel DMB) | 0.8 |
| HEC (Natrosol 250LR) | 0.2 |
| Distilled water | 73.0 |
| Dental Flavour A | 25.0 |
| Titanium Dioxide | 1.0 |

The emulsion was sprayed into a reservoir of aqueous calcium chloride and washed with distilled water as detailed in Example 1.

### Example 3

Dental Flavour encapsulates were prepared by making an emulsion of the following ingredients using a Silverson mixer:

| | Weight % |
|---|---|
| Sodium Alginate (Manucol DM ex Nutrasweet Kelco) | 1.0 |
| HEC (Natrosol 250LR) | 0.5 |
| Distilled water | 68.5 |
| Dental Flavour A* | 30.0 |

| | |
|---|---|
| * Containing 0.1% D&C Red # 17 | |

The alginale Manucol DM (Manucol is a Trade Mark) contains a greater proportion of polymannuronic acid to polyguluronic acid units as compared with Manugel DMB. The emulsion was sprayed into a reservoir of aqueous calcium chloride and washed with distilled water as detailed in Example 1.

### Example 4

Dental flavour encapsulates were prepared by making an emulsion of the following ingredients using a Silverson:

| | Weight % |
|---|---|
| Sodium Alginate (Manugel DMB) | 0.8 |
| PGA*(Kelcoloid S ex NutraSweet Kelco) | 0.4 |
| Distilled water | 72.8 |
| Dental Flavour A | 25.0 |
| Titanium Dioxide | 1.0 |

| | |
|---|---|
| *Propylene Glycol Alginate Sodium Alginate ex NutraSweet Kelco | |

The emulsion was sprayed into a reservoir of aqueous calcium chloride and washed with distilled water as detailed in example 1. The encapsulates were then Incorporated into the toothpaste formulation (T1) in an amount to constitute 1% flavour by weight, using standard toothpaste mixing techniques.

The capsules dosed into the toothpaste formulation (T1) were found to remained intact after storage at 45°C for four months with only slight leaching of the flavour. Stability is improved in the presence of the anionic detergent due to the addition of magnesium sulphate to the product formulation which decrease the critical micelle concentration of the surfactant, resulting in less free surfactant to destabilise the capsule system.

### Toothpaste T1

| | Weight % |
|---|---|
| Sorbitol (70% solution) | 60.6 |
| Water (mains) | 12.15 |
| Sodium monoflunrophosphate | 0.8 |
| Trisodium phosphate anhydrous | 0.1 |
| Magnesium sulphate anhydrous | 2.0 |
| Silica thickener | 8.0 |
| Silica abrasive | 8.0 |
| Sodium carboxymethyl cellulose | 0.55 |
| Empicol LZPV/C | 2.0 |
| Polyethyleneglycol 1500 | 4.0 |
| Saccharin (25% aq) | 0.8 |

Empicol LZPV₁C- ex Albright a wilson Detergents Division

### Example 5

Dental flavour encapsulates were prepared by making an emulsion of the following ingredients using a Silverson:

| | Weight % |
|---|---|
| Sodium Alginate (Manugel DMB) | 0.8 |
| PGA* (Kelcoloid S ex NutraSweet Kelco) | 0.4 |
| Pectin (No. P-2157 ex Sigma) | 0.4 |
| Distilled water | 57.4 |
| Dental Flavour A | 40.0 |
| Titanium Dioxide | 1.0 |

| | |
|---|---|
| *Propylene Glycol Alginate Sodium Alginate ex NutraSweet Kelco | |

The emulsion was sprayed into a reservoir of aqueous calcium chloride and washed with distilled water as detailed in example 1. The combination of gums was found to give stable capsules with flavour loadings as high as 40%.

### Example 6

Dental flavour encapsulates were prepared by making an emulsion of the following ingredients using a Silverson:

| | Weight % |
|---|---|
| Sodium Alginate (Manugel DMB) | 0.4 |
| Gellan Gum (Kelcogel F) | 0.4 |
| Distilled water | 64.0 |
| Colloidal silica (Ludox HS 40) | 10.0 |
| PGA (Kelcoloid S) | 0.2 |
| Dental Flavour A* | 25.0 |

| | |
|---|---|
| * Contaming 0.1% Phthalocyanine Blue 22005 ex Anstead International Sodium Alginate ex NutraSweet Kelco Polyvinyl alcohol supplied by British Traders Gellan Gum (Kelcogel F) ex NutraSweet Kelco Colloidal silica (Ludox) ex Du Pont PGA: Propylene Glycol Alginate(Kelcoloid S ex *NutraSweet* Kelco) | |

The emulsion was sprayed into a reservoir of aqueous calcium chloride and washed with distilled water as detailed in example 1.

Empicol LZPV/C - ex Albright & Wilson Detergents Divison

## Claims

1. A water-based toothpaste comprising: a hydrated dental flavour encapsulate comprising an aqueous emulsion or dispersion of dental flavour in an alginate matrix; an anionic surfactant; and an electrolyte, wherein the electrolyte comprises magnesium sulphate.

2. A toothpaste according to claim 1, wherein the surfactant is sodium lauryl sulphate.

3. A toothpaste according to claim 1 or 2, wherein the dental flavour is present in the encapsulate in an amount up to 60%, preferably 10 to 60%, more preferably 20 to 60%, most preferably 25 to 30%, by weight of the total weight of the encapsulate.

4. A toothpaste according to any one of the preceding claims, wherein the concentration of the electrolyte is in the range from 0.2 to 5%, preferably 0.5 to 3%, and more preferably 1 to 2%, by weight of the total weight of the toothpaste.

5. A toothpaste according to any one of the preceding claims, wherein the alginate is present in the encapsulate in an amount up to 5%, typically about 1%, by weight of the total weight of the encapsulate.

6. A toothpaste according to any one of the preceding claims, wherein the weight ratio of dental flavour to alginate is in the range 5:1 to 300:1, preferably 10:1 to 100:1, more preferably 20:1 to 60:1.

7. A toothpaste according to any one of the preceding claims, wherein the encapsulate further comprises a water soluble polysaccharide, preferably having a molecular weight of greater than 10,000, as an emulsifying agent.

8. A toothpaste according to claim 7, wherein the emulsifying agent is selected from (i) hydroxy cellulosic materials such as hydroxyethyl cellulose, hydroxypropyl cellulose and hydroxypropylmethyl cellulose, and (ii) other polysaccharides such as Pectins, gum Tragacanth, propylene glycol alginate, gum Arabic and gum Ghatti.

9. A toothpaste according to claim 7 or 8, wherein the emulsifying agent is present in the encapsulate in an amount up to about 1%, typically about 0.5%, by weight of the total weight of the encapsulate.

10. A toothpaste according to any one of the preceding claims, wherein the encapsulate has a diameter in the range 0.1 to 3mm, preferably 0.3 to 1mm.

## Patentansprüche

1. Zahnpasta auf Wasserbasis, umfassend ein hydratisiertes Verkapselungsprodukt eines dentalen Aromastoffs, das eine wässrige Emulsion oder Dispersion eines dentalen Aromastoffs in einer Alginatmatrix umfasst, ein anionisches oberflächenaktives Mittel und einen Elektrolyten, wobei der Elektrolyt Magnesiumsulfat umfasst.

2. Zahnpasta nach Anspruch 1, wobei das oberflächenaktive Mittel Natriumlaurylsulfat ist.

3. Zahnpasta nach Anspruch 1 oder Anspruch 2, wobei der dentale Aromastoff im Verkapselungsprodukt in einer Menge von bis zu 60 Gew.%, vorzugsweise von 10 bis 60 Gew.%, bevorzugter von 20 bis 60 Gew.%, am bevorzugtesten von 25 bis 30 Gew.% des Gesamtgewichts des Verkapselungsproduktes vorliegt.

4. Zahnpasta nach einem der vorangehenden Ansprüche, wobei die Konzentration des Elektrolyten im Bereich von 0,2 bis 5 Gew.%, vorzugsweise 0,5 bis 3 Gew.% und bevorzugter 1 bis 2 Gew.% des Gesamtgewichts der Zahnpasta ist.

5. Zahnpasta nach einem der vorangehenden Ansprüche, wobei das Alginat im Verkapselungsprodukt in einer Menge von bis zu 5 Gew.%, typischerweise von etwa 1 Gew.% der Gesamtmenge des Verkapselungsproduktes vorliegt.

6. Zahnpasta nach einem der vorangehenden Ansprüche, wobei das Gewichtsverhältnis von dentalem Aromastoff zu Alginat im Bereich von 5:1 bis 300:1, vorzugsweise von 10:1 bis 100:1, bevorzugter von 20:1 bis 60:1 liegt.

7. Zahnpasta nach einem der vorangehenden Ansprüche, wobei das Verkapselungsprodukt außerdem ein wasserlösliches Polysaccharid, das vorzugsweise ein Molekulargewicht von größer als 10.000 hat, als Emulgiermittel umfasst.

8. Zahnpasta nach Anspruch 7, wobei das Emulgiermittel aus
(i) Hydroxycellulosematerialien, z.B. Hydroxyethylcellulose, Hydroxypropylcellulose und Hydroxypropylmethylcellulose, und
(ii) anderen Polysacchariden, z.B. Pectinen, Tragantgummi, Propylenglykolalginat, Gummiarabicum und Ghatti-Gummi ausgewählt ist.

9. Zahnpasta nach Anspruch 7 oder 8, wobei das Emulgiermittel in dem Verkapselungsprodukt in einer Menge von bis zu etwa 1 Gew.%, typischerweise etwa 0,5 Gew.% des Gesamtgewichts des Verkapselungsproduktes vorliegt.

10. Zahnpasta nach einem der vorangehenden Ansprüche, wobei das Verkapselungsprodukt einen Durchmesser im Bereich von 0,1 bis 3 mm, vorzugsweise 0,3 bis 1 mm hat.

## Revendications

1. Dentifrice à base hydrique comprenant : un encapsulat d'arôme dentaire hydraté comprenant une dispersion ou émulsion aqueuse d'arôme dentaire dans une matrice d'alginate ; un tensio-actif anionique ; et un électrolyte, dans lequel l'électrolyte comprend du sulfate de magnésium.

2. Dentifrice selon la revendication 1, dans lequel le tensio-actif est du laurylsulfate de sodium.

3. Dentifrice selon la revendication 1 ou 2, dans lequel l'arôme dentaire est présent dans l'encapsulat en une quantité allant jusqu'à 60 %, de préférence de 10 à 60 %, de manière préférée de 20 à 60 %, de manière préférée entre toutes de 25 à 30 %, en poids du poids total de l'encapsulat.

4. Dentifrice selon l'une quelconque des revendications précédentes, dans lequel la concentration de l'électrolyte se situe dans l'intervalle de 0,2 à 5 %, de préférence de 0,5 à 3 %, et de manière préférée de 1 à 2 %, en poids du poids total du dentifrice.

5. Dentifrice selon l'une quelconque des revendications précédentes, dans lequel l'alginate est présent dans l'encapsulat en une quantité allant jusqu'à 5 %, généralement environ 1 %, en poids du poids total de l'encapsulat.

6. Dentifrice selon l'une quelconque des revendications précédentes, dans lequel le rapport de poids de l'arôme dentaire sur l'alginate se situe dans l'intervalle de 5:1 à 300:1, de préférence de 10:1 à 100:1, de manière préférée de 20:1 à 60:1.

7. Dentifrice selon l'une quelconque des revendications précédentes, dans lequel l'encapsulat comprend en outre un polysaccharide soluble dans l'eau, ayant de préférence un poids moléculaire supérieur à 10000, comme agent émulsifiant.

8. Dentifrice selon la revendication 7, dans lequel l'émulsifiant est choisi parmi (i) des matériaux hyroxy cellulosiques comme l'hyroxyéthylcellulose, l'hydroxypropylcellulose et l'hydroxypropylméthylcellulose, et (ii) d'autres polysaccharides comme les pectines, la gomme adragante, l'alginate de propylène glycol, la gomme arabique et la gomme ghatti.

9. Dentifrice selon la revendication 7 ou 8, dans lequel l'agent émulsifiant est présent dans l'encapsulat en une quantité allant jusqu'à environ 1 %, généralement environ 0,5 %, en poids du poids total de l'encapsulat.

10. Dentifrice selon l'une quelconque des revendications précédentes, dans lequel l'encapsulat a un diamètre compris dans l'intervalle de 0,1 à 3 mm, de préférence de 0,3 à 1 mm.
